Europäisches Patentamt

European Patent Office

Office européen des brevets

(19)

(11) Veröffentlichungsnummer: **0 010 581**
**B1**

(12) **EUROPÄISCHE PATENTSCHRIFT**

(21) Anmeldenummer: **79102970.5**

(22) Anmeldetag: **16.08.79**

(51) Int. Cl.³: **C 07 C  69/44,**
**C 07 C  69/533,**
**C 07 C  67/38, C 07 C  67/48**

(54) Verfahren zur Herstellung von Butandicarbonsäuredimethylestern.

(30) Priorität: **30.08.78 DE 2837815**

(43) Veröffentlichungstag der Anmeldung:
**14.05.80 Patentblatt 80/10**

(45) Bekanntmachung des Hinweises auf die Patenterteilung:
**11.02.81 Patentblatt 81/6**

(84) Benannte Vertragsstaaten:
**BE CH DE FR GB IT NL**

(56) Entgegenhaltungen:
**FR - A - 2 384 738**

(73) Patentinhaber: **BASF Aktiengesellschaft**
**Carl-Bosch-Strasse 38**
**D-6700 Ludwigshafen (DE)**

(72) Erfinder: **Kummer, Rudolf, Dr.**
**Kreuzstrasse 6**
**D-6710 Frankenthal 5 (DE)**
(72) Erfinder: **Schneider, Heinz-Walter, Dr.**
**Bruesseler Ring 43**
**D-6700 Ludwigshafen (DE)**
(72) Erfinder: **Weiss, Franz-Josef, Dr.**
**Schlehdornweg 69**
**D-6940 Weinheim (DE)**
(72) Erfinder: **Leman, Otto**
**Im Wandelgarten 8**
**D-6719 Weisenheim (DE)**

Courier Press, Leamington Spa, England.

EP 0 010 581 B1

Verfahren zur Herstellung von Butandicarbonsäuredimethylestern

Gegenstand der Erfindung ist ein Verfahren zur Herstellung von Butandicarbonsäuredimethylestern, bei dem man Butadien oder Butadien enthaltende Kohlenwasserstoffgemische mit Kohlenmonoxid und Methanol in Gegenwart von tertiären Stickstoffbasen und Kobaltcarbonylkatalysatoren umsetzt und anschließend nach Abtrennen des größten Teils der tertiären Stickstoffbasen den so erhaltenen Pentensäuremethylester mit Methanol in Gegenwart des im Reaktionsgemischs verbleibenden Katalysators zu Butandicarbonsäuredimethylester umsetzt.

Aus der DAS 20377 82 ist ein Verfahren zur Herstellung von Adipinsäure bekannt, bei dem man Butadien, Kohlenmonoxid und Wasser unter erhöhtem Druck in Gegenwart von Rhodiumverbindungen als Katalysatoren umsetzt. Die hierbei erzielten Ausbeuten ermutigen jedoch nicht, das Verfahren technisch durchzuführen. Nach einem anderen in der DAS 1 518 216 beschriebenen Verfahren wird Butadien in Gegenwart von Kobaltcarbonyl und Pyridin und Wasser unter 430 bar und bei 210 °C zu Dicarbonsäuren umgesetzt. Die hierbei erzielte Ausbeute beträgt 50—70% d.Th. bezogen auf Butadien. Ferner ist aus Bull. Chem. Soc. of Japan, Band 46, 1973, Seiten 24—35 bekannt, daß man Adipinsäuredimethylester erhält, durch Umsetzen von Butadien mit Kohlenmonoxyd und Methanol in Gegenwart von Kobaltcarbonyl und Pyridin und anschließende Carbonylierung des so erhaltenen Pentensäuremethylesters mit demselben Katalysator, wobei in der zweiten Stufe die Temperatur auf 240°C erhöht wird. Die Ausbeuten an Adipinsäuredimethylester liegen jedoch nur zwischen 47 und 51%. Insbesondere ist bei allen Verfahren auch die niedrige Reaktionsgeschwindigkeit von Butadien zum Pentensäuremethylester nachteilig.

Es war deshalb die technische Aufgabe gestellt, die Reaktionsgeschwindigkeit bei der Carbonylierung von Butadien zu Pentensäureester zu erhöhen, ohne jedoch die Schritte der Katalysatorzubereitung sowie die nachfolgenden Schritte der Carbonylierung von Pentensäureester zu Adipinsäureester bzw. die Wiedergewinnung des Katalysators mit erhöhten Katalysatormengen zu belasten.

Diese Aufgabe wird gelöst in einem Verfahren zur Herstellung von Butandicarbonsäuredimethylester bei dem man

a) Butadien oder Butadien enthaltende Kohlenwasserstoffgemische mit Kohlenmonoxid und Methanol in Gegenwart von tertiären Stickstoffbasen und Kobaltcarbonylkatalysatoren bei Temp. von 80 bis 150°C unter erhöhtem Druck umsetzt.

b) anschließend den größeren Teil der tertiären Stickstoffbasen sowie überschüssige Kohlenwasserstoffe abtrennt und

c) dann den erhaltenen Pentensäuremethylester mit Kohlenmonoxid und Methanol in Gegenwart der verbleibenden Menge an tertiären Stickstoffbasen und Kobaltcarbonylkatalysatoren beim Temperaturen von 140 bis 200°C unter erhöhtem Druck zu Butandicarbonsäuremethylester umsetzt, dadurch gekennzeichnet, daß man das in der Stufe a) nach Umsetzung des Butadiens erhaltene Reaktionsgemisch soweit abkühlt, daß es sich in zwei Phasen trennt und ein Teilstrom von 20—80 Vol.-% der spezifisch schwereren Phase in die Stufe a) zurückführt.

Das neue Verfahren hat den Vorteil, daß die Carbonylierung von Butadien schneller verläuft, wobei jedoch die übrigen Stufen nicht durch einen höheren Kobaltgehalt belastet werden.

Man geht von reinem Butadien-1.3 oder Butadien enthaltenden Kohlenwasserstoffgemischen aus, Solche Kohlenwasserstoffgemische enthalten z.B. neben Butadien gesättigte Kohlenwasserstoff mit 3—5 Kohlenstoffatomen und einfach olefinisch ungesättigte Kohlenwasserstoffe mit 3—5 Kohlenstoffatomen. Der Butadiengehalt soll in der Regel mehr als 10 Gew.-% betragen. In der Technik werden insbesondere $C_4$-Schnitte als Ausgangsgemisch verwendet. Als solche seien alle Gemische von überwiegend unverzweigten $C_4$-Kohlenwasserstoffen definiert, die mehr als 10 Gew.-% Butadien-1.3 (Butadien und mehr als 15 Gew.-% Butene) enthalten. Je nach Provenienz liegen die einzelnen Komponenten solcher Gemische in folgenden Anteilen vor:

| Butadien | 40—60 Gew.-% |
|---|---|
| Isobuten | 20—35 Gew.-% |
| Buten-1 | 10—25 Gew.-% |
| Buten-2 | 5—15 Gew.-% |
| Butane | 1—10 Gew.-% |
| Butine | 0,1—3 Gew.-% |

Solche $C_4$-Schnitte fallen beispielsweise an, bei der Dehydrierung von Butan oder Buten oder als Nebenprodukte bei der Äthylengewinnung durch thermische Spaltung (cracken) von Leichtbenzin (Naphthan) oder höheren Kohlenwasserstoffschnitten.

Die Umsetzung erfolgt mit Methanol vorteilhaft im Überschuß, insbesondere von 1,5 bis 5 Mol Methanol je Mol Butadien.

Vorzugsweise führt man die Umsetzung bei Temperaturen von 120—140°C und unter Drücken von 600 bis 1200 bar durch. Je Mol Butadien verwendet man in der Regel 0,05—0,15 g Atom Kobalt in Form von Kobaltcarbonylkomplexen. Kohlenmonoxid wird vorteilhaft im Überschuß, z.B. dem 1,5 bis 10 fachen der stöchiometrisch erforderlichen Menge angewandt.

Geeignete tertiäre Stickstoffbasen haben vorteilhaft einen $Pk_a$-Wert von 3—11, mit der Maßgabe, daß die tertiären Stickstoffbasen vorzugsweise niedriger sieden als der herzustellende Pentensäuremethylester. Vorzugsweise verwendet man N-heterocyclische Verbindungen wie Pyridin ($Pk_a$ 5,3) Methylpyridine wie 3-Picolin ($Pk_a$ 6,0), Isochinolin ($Pk_a$ 5,4) ferner Trialkylamine wie Trimethylamin ($Pk_a$ 9,8) oder Triäthylamin ($Pk_a$ 11,0). Besondere technische Bedeutung hat Pyridin erlangt. Besonders bewährt hat es sich, wenn man je Mol Kobaltcarbonylkatalysator 5 bis 50 Mol Pyridin anwendet.

Die in der Stufe a) verwendeten Kobaltkatalysatoren bringt man vorteilhaft bereits als Kobaltcarbonyl, insbesondere gelöst im Butadien oder $C_4$-Schnitt in das Gemisch ein. Eine solche Lösung erhält man beispielsweise durch Umsetzen einer wäßrigen Lösung von fettsauren Kobaltsalzen wie Acetaten oder Butyraten mit einem Gemisch aus Kohlenmonoxid und Wasserstoff in Gegenwart von Aktivkohle bei einer Temp. von 100 bis 170°C unter einem Druck von 100 bis 400 bar. Aus der wäßrigen Lösung wird das entstandene Kobaltcarbonyl dann mit Butadien oder $C_4$-Schnitt extrahiert.

Das in der Stufe a) erhaltene Reaktionsgemisch enthält neben nicht umgesetztem Butadien ggf. andere Kohlenwasserstoffe, tertiäre Stickstoffbasen, Kobaltcarbonylkatalysatoren, überschüssiges Methanol, den als Wertprodukt erzeugten Pentensäuremethylester sowie Nebenprodukte wie Valeriansäureester Butylketone sowie Polymerisate des Butadiens.

Erfindungsgemäß kühlt man das aus der Stufe a) erhaltene Reaktionsgemisch soweit ab, daß es sich in zwei Phasen trennt. Man erhält hierbei eine spezifisch leichtere Oberphase aus den restlichen eingesetzten Kohlenwasserstoffen sowie jeweils bezogen auf den gesamten Reaktionaustrag 70 bis 95 Gew.-% des Pentensäuremethylesters, 60 bis 85 Gew.-% der Stickstoffbase, 10 bis 40 Gew.-% des Methanols, 1 bis 4 Gew.-% des Kobaltcarbonylkatalysators. Ferner erhält man eine spezifisch schwerere Unterphase, die praktisch den gesamten Katalysator (96 bis 99 Gew.-%), geringe Mengen (5 Gew.-%) der Kohlenwasserstoffe, 5 bis 30 Gew.-% des Pentensäuremethylesters, 15 bis 40 Gew.-% der Stickstoffbasen und 60 bis 90 Gew.-% des Methanols enthält. Die Trennung der Phasen erfolgt auf übliche Weise, z.B. durch dekantieren.

Für die Phasentrennung hat es sich als günstig erwiesen, wenn ein Molverhältnis von Pentensäuremethylester zu Methanol von 1:005 bis 1:0,5 eingehalten wird. Vorteilhaft kühlt man das Gemisch auf eine Temperatur von —10 bis +40°C, insbesondere von —5 bis +20°C ab.

Außerdem ist es vorteilhaft, wenn der Kobaltcarbonylkatalysator nicht mehr als Allylkomplex vorliegt. Dies erreicht man beispielsweise dadurch, daß man das Reaktionsgemisch vor der Trennung 5 bis 60 Minuten bei einer Temp. von 100 bis 160°C und einem Druck von 5 bis 200 bar mit Kohlenmonoxid behandelt.

Aus der spezifisch schwereren Unterphase wird ein Teilstrom von 20—80 Vol.%, insbesondere 50 bis 70 Vol.-% in die Stufe a) zurückgeführt. Es versteht sich, daß die vorbeschriebenen, Reaktionsbedingungen für die Stufe a) eingestellt werden.

Aus der spezifisch leichteren Oberphase werden die Kohlenwasserstoffe sowie der größte Teil des Pyridins abgetrennt, z.B. durch Destillation.

Der verbleibende Teil der Oberphase wird mit dem restlichen Teil der Unterphase gemischt und in der Stufe c) umgesetzt. Hierbei hält man vorteilhaft ein Molverhältnis von Pentensäuremethylester zu Methanol von 1:1,5 bis 4 ein. Es wird eine Temperatur von 140 bis 200°C, insbesondere von 150 bis 180°C angewandt. Vorteilhaft wendet man Drücke von 100 bis 400 bar an. Die Umsetzung erfolgt mit Kohlenmonoxid, dem man zur Erhöhung der Reaktionsgeschwindigkeit vorteilhaft einige Volumen-% Wasserstoff, z.B. 0,2 bis 4 Vol.-% zusetzt. Ferner hat es sich als vorteilhaft erwiesen, wenn je Mol Kobaltkatalysator 2 bis 10 Mol tertiäre Stickstoffbasen sowie je Mol Pentinsäuremethylester 0,01 bis 0,08 Mol Kobaltcarbonylkomplex im Reaktionsgemisch vorhanden sind. Das erhaltene Reaktionsgemisch wird beispielsweise wie folgt aufgearbeitet:

Nach Entspannen des in der Stufe c) erhaltenen Reaktionsgemisches werden überschüssiges Methanol und freie tertiäre Stickstoffbasen abdestilliert. Die chemisch an den Katalysator gebundenen tertiären Stickstoffbasen (1 bis 2 Mol pro g Atom Kobalt) werden dabei nicht abdestilliert. Um eine Zersetzung des Kobaltkomplexes unter Abscheiden von metallischem Kobalt zu vermeiden, hat es sich als zweckmäßig erwiesen, in den Sumpf der Kolonne einen langsamen Strom von Kohlenmonoxid oder Kohlenmonoxid enthaltenden Gasen einzuleiten.

Das verbleibende Kobaltkatalysator, Butandicarbonsäuremethylester und Nebenprodukte enthaltende Reaktionsgemisch wird zunächst mit Oxidationsmitteln wie molekularen Sauerstoff oder solchen enthantende Gase, insbesondere Luft, im wäßrig sauren Medium vorteilhaft bei einem pH-Wert von 3 bis 6, bei Temperaturen von 80 bis 160°C behandelt. Nach der Behandlung trennt man z.B. durch Dekantieren in eine organische und wäßrige Phase. Aus der organischen Phase erhält man durch fraktionierte Destillation restliche tertiäre Stickstoffbasen, nicht umgesetzten Pentensäuremethylester, der wieder der Carbonylierung zugeführt wird sowie ein Gemisch aus Butandicarbonsäuredimethylestern (80 bis 85 Gew.-% Adipinsäuredimethylester, 11 bis 15 Gew.-% 2-Methylglutarsäuredimethylester, 3 bis 6 Gew.-% 2-Äthylbernsteinsäuredimethylester). Das Estergemisch kann zur Herstellung von Diolen oder Polyestern verwendet werden. Der aus dem Estergemisch durch fraktionierte Destillation erhältliche Adipinsäuredimethylester eignet sich zur Herstellung von Adipinsäure.

Die Kobaltsalze, und freie Säure enthaltende wäßrige Phase wird vorteilhaft weider als Ausgangslösung zur Herstellung von Kobaltcarbonyl zurückgeführt.

Das Verfahren nach der Erfindung sei im folgenden Beispiel veranschaulicht:

Beispiel 1

a) In einem Hochdruckgefäß mit 0,5 l Inhalt, setzt man ein Gemisch aus 135,2 g C₄-Schnitt mit einem Gehalt von 40% (m/m) Butadien-1.3 (=1,0 mol Butadien-1.3) 79,1 g (1,0 mol) Pyridin, 38,5 g (1,2 mol) Methanol und 0,04 mol Kobalt pro Mol Butadien-1.3 in Form von Kobaltcarbonyl mit Kohlenmonoxid bei 135°C und 900 bar um. Die Reaktionszeit beträgt 110 Minuten.

b) Nach Beendigung der Reaktion kühlt man auf −5°C ab und entspannt vorsichtig die Gasphase. Im Abgas findet man kein Butadien-1.3, d.h. der Umsatz ist quantitativ. Durch die Abkühlung zerfällt der Reaktorinhalt in 2 Phasen. Es bilden sich 224 g einer spezifisch leichteren Phase (Oberphase), welche die folgenden prozentualen Anteile des Reaktoraustrages enthält:

2,2 Gew.-% (m/m) des Kobalts

70 „ „ des Pyridins

82 „ „ des Pentenesters

45 „ „ des Methanols

95 „ „ des C₄-Schnittes

Die spezifisch schwerere Phase (Unterphase) mit einer Menge von 64 g enthält folgende Anteile des Reaktoraustrages:

97,8 Gew.-% (m/m) des Kobalts

18 „ „ des Pentenesters

30 „ „ des Pyridins

55 „ „ des Methanols

5 „ „ des C₄-Schnittes

c) Aus obiger Analyse geht hervor, daß sich praktisch das gesamte, in Punkt a) eingesetzte Kobalt in der Unterphase befindet. Man führt 70 Vol.-% der spezifisch schwereren Unterphase (entsprechend etwa 70% der Kobaltmenge) in die Stufe a) zurück und gibt frisches Kobalt in Form von Kobaltcarbonyl zu, so daß sich ein Verhältnis von Butadien-1.3 : Kobalt von 1:0,1 einstellt, welches dem 2,5 fachen unter Punkt a) beschriebenen Verhältnis entspricht.

d) Stellt man die Mc verhältnisse der übrigen an der Reaktion beteiligten Komponenten wie in Stufe a) ein und carbonyliert bei 135°C und 900 bar, so ergibt sich eine Reaktionszeit von 55 Minuten; d.h. die Reaktionsgeschwindigkeit hat sich um den Faktor 2 erhöht.

**Patentansprüche**

1. Verfahren zur Herstellung von Butandicarbonsäuredimethylestern, bei dem man

a) Butadien oder Butadien enthaltende Kohlenwasserstoffgemische mit Kohlenmonoxid und Methanol in Gegenwart von tertiären Stickstoffbasen und Kobaltcarbonylkatalysatoren bei Temperaturen von 80 bis 150°C unter erhöhtem Druck umsetzt

b) den größten Teil der tertiären Stickstoffbasen sowie ggf. überschüssige Kohlenwasserstoffe abtrennt und

c) den so erhaltenen Pentensäuremethylester in Gegenwart von Kobaltcarbonylkatalysatoren und der restlichen Menge an tertiären Stickstoffbasen mit Kohlenmonoxid und Methanol bei Temperaturen von 140 bis 200°C unter erhöhtem Druck zu Butandicarbonsäuredimethylester umsetzt, *dadurch gekennzeichnet,* daß man das in der Stufe a) nach Umsetzung des Butadiens erhaltene Reaktionsgemisch soweit abkühlt, daß es sich in zwei Phasen trennt und einen Teilstrom von 20 bis 80 Vol.-% der spezifisch schwereren Unterphase in die Stufe a) zurückführt.

2. Verfahren nach Anspruch 1, *dadurch gekennzeichnet,* daß man das Reaktionsgemisch aus der Stufe a) auf eine Temperatur von −10°C bis +40°C abkühlt.

3. Verfahren nach Anspruch 1 und 2, *dadurch gekennzeichnet,* daß man bei der Phasentrennung ein Molverhältnis von Pentensäuremethylester zu Methanol von 1:0,05 bis 1:0,5 einhält.

**Revendications**

1. Procédé de préparation d'esters diméthyliques d'acides butanedicarboxyliques dans lequel

a) on fait réagir, à des températures comprises entre 80 et 150°C, sous pression, du butadiène ou des mélanges d'hydrocarbures contenant du butadiène avec de l'oxyde de carbone et du méthanol, en présence de bases d'azote tertiaires et de catalyseurs de cobalt carbonyle,

b) on sépare la majeure partie des bases d'azote tertiaires et, le cas échéant, des hydrocarbures en excès, et

c) on fait réagir l'ester méthylique d'acide penténique ainsi obtenu, en présence de catalyseurs de cobalt carbonyle et de la quantité restante de bases d'azote tertiaires, avec de l'oxyde de carbone et du méthanol, à des températures comprises entre 140 et 200°C, sous pression, pour obtenir l'ester diméthylique d'acide butanedicarboxylique,

ce procédé étant caractérisé par le fait que l'on refroidit le mélange réactionnel obtenu dans le stade a) après réaction du butadiène, à la température nécessaire pour qu'il se sépare en deux phases et on ramène au stade a) un courant partiel représentant de 20 à 80% en volume de la phase inférieure spécifiquement plus lourde.

2. Procédé selon la revendication 1, dans lequel on refroidit le mélange réactionnel venant du stade a) à une température comprise entre −10 et +40°C.

3. Procédé selon l'une des revendications 1 et 2, dans lequel on maintient, lors de la séparation des phases, un rapport molaire, ester méthylique d'acide penténique/méthanol, compris entre 1/0,05 et 1/0,5.

## Claims

1. A process for the preparation of dimethyl butanedicarboxylates, wherein

a) butadiene or a hydrocarbon mixture containing butadiene is reacted with carbon monoxide and methanol in the presence of a tertiary nitrogen base and a cobalt carbonyl catalyst at from 80 to 150°C under superatmospheric pressure,

b) the greater part of the tertiary nitrogen base and any excess hydrocarbons are removed and

c) the methyl pentenoate thus obtained is reacted with carbon monoxide and methanol, in the presence of a cobalt carbonyl catalyst and the residual amount of tertiary nitrogen base, at from 140 to 200°C under superatmospheric pressure, to give the dimethyl butanedicarboxylate,

*characterized in that* the reaction mixture obtained in stage a) after reaction of the butadiene is cooled until it separates into two phases, and a part-stream of from 20 to 80% by volume of the lower phase of higher specific gravity is recycled to stage a).

2. A process as claimed in claim 1, *characterized in that* the reaction mixture from stage a) is cooled to from −10°C to +40°C.

3. A process as claimed in claims 1 and 2, *characterized in that* a molar ratio of methyl pentenoate to methanol of from 1:0.05 to 1:0.5 is maintained in the phase separation.